(19) Europäisches Patentamt
European Patent Office
Office européen des brevets

(11) Veröffentlichungsnummer: **0 055 973**
**B1**

(12) # EUROPÄISCHE PATENTSCHRIFT

(45) Veröffentlichungstag der Patentschrift: **23.01.85**

(51) Int. Cl.⁴: **C 07 D 405/04**, D 06 L 3/12

(21) Anmeldenummer: **81810512.4**

(22) Anmeldetag: **21.12.81**

(54) **Benzimidazole.**

(30) Priorität: **29.12.80 CH 9611/80**
**16.07.81 CH 4666/81**

(43) Veröffentlichungstag der Anmeldung: **14.07.82 Patentblatt 82/28**

(45) Bekanntmachung des Hinweises auf die Patenterteilung: **23.01.85 Patentblatt 85/4**

(84) Benannte Vertragsstaaten: **CH DE FR GB IT LI**

(56) Entgegenhaltungen:
**DE - A - 1 469 227**
**DE - A - 2 159 469**
**DE - A - 2 346 316**
**DE - A - 2 853 765**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Postfach, CH-4002 Basel (CH)**

(72) Erfinder: **Meyer, Hans Rudolf, Dr., Bollwerkstrasse 102, CH-4102 Binningen (CH)**

**EP 0 055 973 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft neue am Benzoring unsubstituierte Benzimidazole, Verfahren zu deren Herstellung sowie deren Verwendung zum optischen Aufhellen von organischen Materialien.

Aus der DE-OS 1 469 227, 2 031 774 und 2 159 469 sind die besonders leicht zugänglichen, am Benzoring des Benzimidazolrestes unsubstituierten Benzimidazole bekannt, welche aber unbefriedigende Aufhelleffekte ergeben. Aus den DE-OS 1 469 227, 2 346 316 und 2 853 765 sind nicht quaternierte und aus den DE-OS 1 469 227 und 2 159 469 quaternierte Benzimidazole bekannt, welche keine hohen Maximaleffekte bzw. keine hohen Aufhelleffekte erreichen.

Der vorliegenden Erfindung lag deshalb die Aufgabe zugrunde, am Benzoring des Benzimidazolrestes unsubstituierte Benzimidazole zu finden, welche diese Mängel nicht aufweisen.

Es wurde nun gefunden, daß sich mit einigen ausgewählten, am Benzoring des Benzimidazolrestes unsubstituierten Benzimidazolen, überraschend hohe Aufhelleffekte erzielen lassen.

Die erfindungsgemäßen neuen Benzimidazole entsprechen der Formel

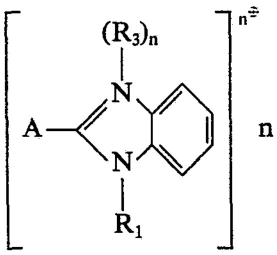 n $X^{\ominus}$ (1)

worin

A den Rest

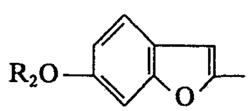 oder 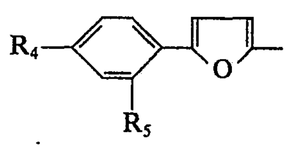

| | |
|---|---|
| $R_1$ | Cyanomethyl, 1-Cyanoäthyl oder, wenn n die Zahl 1 ist, auch 2-Cyanoäthyl oder $C_{3-6}$-Carbalkoxymethyl, |
| $R_2$ | $C_{1-4}$-Alkyl oder $C_{3-4}$-Alkenyl, |
| $R_3$ | Cyanomethyl, Cyanoäthyl oder $C_{3-6}$-Carbalkoxymethyl, wobei wenn $R_3$ Cyanoäthyl bedeutet, $R_1$ ist 1-Cyanoäthyl oder 2-Cyanoäthyl, |
| $R_4$ und $R_5$ | unabhängig voneinander Wasserstoff oder Chlor |
| $X^{\ominus}$ | ein farbloses Anion einer organischen oder anorganischen Säure und |
| n | die Zahl 0 oder 1 |

bedeuten.

Unter »$C_{3-6}$-Carbalkoxymethyl« sind die Reste $C_4H_9OOCCH_2-$, $C_3H_7OOCCH_2-$, deren Isomere und besonders $C_2H_5OOCCH_2-$ und $CH_3OOCCH_2-$ zu verstehen.

Verbindungen der Formel (1), worin n = 0 ist, können auch als Säureaddukte vorliegen und zum Aufhellen verwendet werden.

Als Anion $X^{\ominus}$ kommt jedes farblose Anion einer organischen oder anorganischen Säure in Frage. Seine Natur hat auf die Aufhelleigenschaften der erfindungsgemäßen Verbindungen keinen wesentlichen Einfluß. Das Anion wird in der Regel durch den Herstellungsprozeß (Quaternierung oder Protonierung) eingeführt, doch kann es nach bekannten Methoden (siehe z. B. Houben-Weyl, Methoden der organischen Chemie, Band XL/2, Seiten 620—626) auch durch ein anderes ersetzt werden. Halogenanionen können gemäß DOS 2 549 436 auch durch Anionen aliphatischer Carbonsäuren ausgetauscht werden, indem man das Halogenid in Gegenwart dieser Carbonsäuren mittels Epoxiden als Halogenwasserstoffakzeptoren umsetzt.

Im Rahmen der Verbindungen der Formel (1) sind hervorzuheben solche der Formel

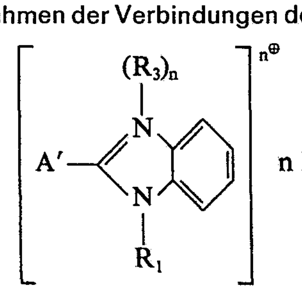 n $X^{\ominus}$ (2)

worin

A' den Rest

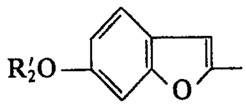 oder 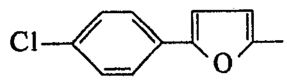 und

$R'_2$ $C_{1-4}$-Alkyl bedeuten und

$R_1$, $R_3$, $X^{\ominus}$ und n die oben angegebene Bedeutung haben.

$R'_2$ ist vorzugsweise Methyl.

Besondere Erwähnung im Rahmen der Verbindungen der Formeln (1) und (2) verdienen jene der Formel

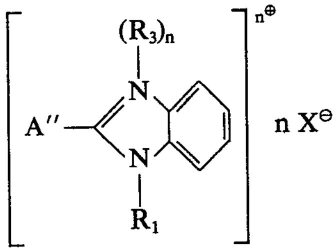 (3)

worin

A'' den Rest

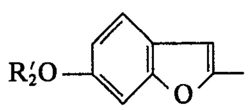

oder, wenn n die Zahl 1 ist, auch den Rest

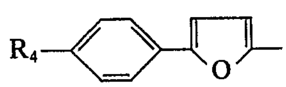

bedeutet, jene der Formel

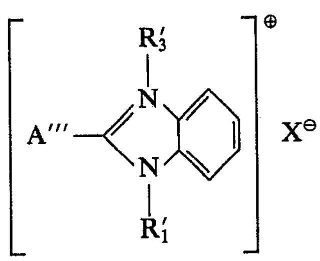 (4)

worin

A''' den Rest

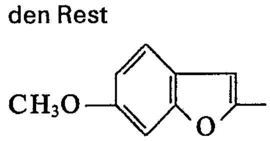 oder 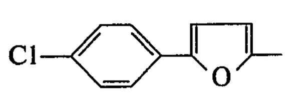

$R'_1$ und $R'_3$ unabhängig voneinander Cyanomethyl, Cyanoäthyl oder $C_{3-6}$-Carbalkoxymethyl bedeuten, wobei wenn $R'_3$ Cyanoäthyl bedeutet, $R'_1$ ist 2-Cyanoäthly, in welchen Formeln $R_1$, $R'_2$, $R_3$, $R_4$, $X^{\ominus}$ und n die oben angegebene Bedeutung haben, sowie jene der Formel

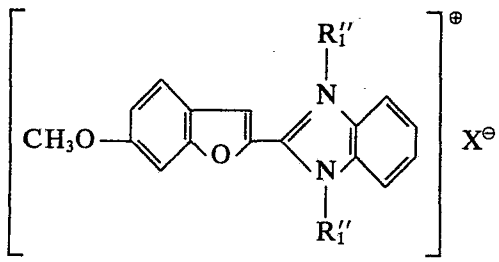
(5)

worin

$R''_1$ Cyanoäthyl oder $C_{3-4}$-Carbalkoxymethyl bedeutet und
$X^\ominus$ die oben angegebene Bedeutung hat.

$R'_1$ ist vorzugsweise identisch mit $R'_3$.

Von praktischem Interesse sind die Verbindungen der Formel

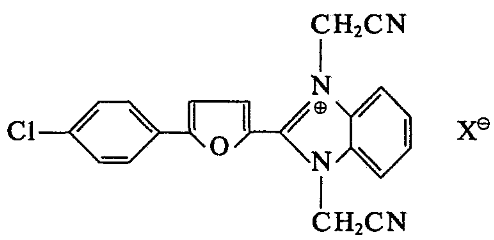
(6)

worin $X^\ominus$ ein farbloses Anion einer organischen oder anorganischen Säure bedeutet,

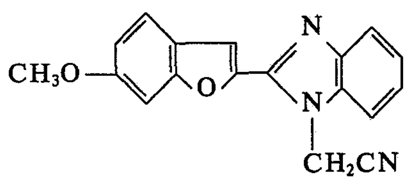
(7)

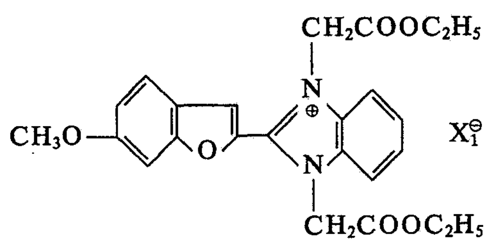
(8)

worin $X_1$ Chlor oder Brom bedeutet, und

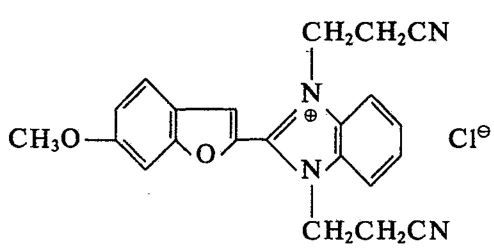
(9)

Die Benzimidazole der Formel (1) werden hergestellt, indem man ein Benzimidazol der Formel

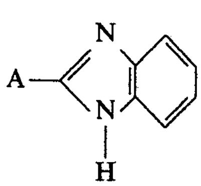
(10)

in einer ersten Stufe in Gegenwart einer Base mit mindestens einem Moläquivalent eines Alkylisierungsmittels $R_1X$ oder mit Acrylnitril in ein Benzimidazol der Formel

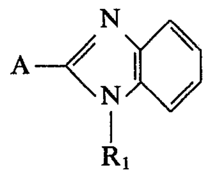

(11)

überführt und dieses gewünschtenfalls in einer zweiten Phase, ohne oder unter vorausgehender Isolierung, mit einer Verbindung der Formel $R_3X$ quaterniert, wobei in den obigen Formeln A, $R_1$, $R_3$ und X die oben angegebenen Bedeutungen haben.

Bei Verbindungen der Formel (1), worin $R_1 = R_3$ ist, kann die Alkylierung und die Quaternierung vorteilhaft gleichzeitig erfolgen, d. h. ohne Isolierung der Verbindung der Formel (11).

Geeignete Basen sind vor allem Alkali- und Erdalkalisalze schwacher Säuren wie Natriumcarbonat, Kaliumcarbonat, Calciumcarbonat oder Erdalkalioxide wie Magnesiumoxid in fein verteilter Form. Auch schwer quaternierbare tertiäre Amine wie Triisopropanolamin oder 2,6-Di-tert.-butylpyridin kommen als Säureakzeptoren in Betracht.

In besonders vorteilhafter Weise verfährt man so, daß das am Stickstoff unsubstituierte Benzimidazol der Formel (10) mit starken Alkalien, wie Alkalihydroxiden oder -alhoholaten, z. B. Natrium- oder Kaliumhydroxid oder -äthylat zunächst in die entsprechenden N-Alkalisalze überführt und diese alsdann alkyliert.

Die Alkylierung bzw. Quaternierung erfolgt vorteilhaft direkt durch Erwärmen in überschüssigem Alkylierungsmittel oder in einem inerten Lösungsmittel bei Temperaturen zwischen 20 und 150° C, vorzugsweise 50 und 140° C, je nach Reaktionsfähigkeit des verwendeten Alkylierungsmittels.

Als Reaktionsmedien, in denen die Quaternierung vorgenommen werden kann, eignen sich im allgemeinen alle inerten Lösungsmittel. Bevorzugt sind solche, die das Ausgangsprodukt lösen und aus denen sich das Endprodukt sofort ausscheidet. Beispielsweise seien genannt: Aromatische Kohlenwasserstoffe wie Benzol, Toluol und Xylol; Halogenkohlenwasserstoffe wie Trichloräthan, Tetrachloräthylen, Chlorbenzol oder Dichlorbenzol, ferner auch Nitroverbindungen wie Nitromethan, Nitropropan, Nitrobenzol, Alkanole und offene oder cyclische Äther wie Butanol, Dibutyläther, Äthylenglykol, Äthylenglykolmonomethyläther, Äthylenglykolmonoäthyläther, Anisol oder Dioxan; Ketone wie Cyclohexanon oder Methyläthylketon; Fettsäureamide wie Dimethylformamid oder Dimethylacetamid; Sulfoxyde wie Dimethylsulfoxyd und Carbonsäureester wie Essigester oder Essigsäure-butylester.

Quaternäre Verbindungen der Formal (1), d. h. solche worin n = 1 ist, werden auch durch Umsetzung von äquivalenten Mengen eines Cumarilsäurehalogenides oder eines Phenyl-furan-carbonsäurehalogenides mit N,N'-disubstituierten o-Phenylendiaminen wie z. B. N,N'-Di-cyanoalkyl- und N,N'-Dicarbalkoxyalkyl-o-phenylendiaminen erhalten. Dieses Verfahren ist besonders geeignet für Verbindungen der Formel (1) worin $R_1$ und $R_3$ 2-Cyanoäthyl bedeuten.

Die Reaktion wird zweckmäßig in inerten Lösungsmitteln wie oben angegeben und/oder in Gegenwart tertiärer Basen wie Pyridin oder Triäthylamin bei Temperaturen zwischen 20 und 150° C, vorzugsweise 50 und 140° C durchgeführt. Nach diesem Verfahren werden z. B. die Verbindungen der Formel

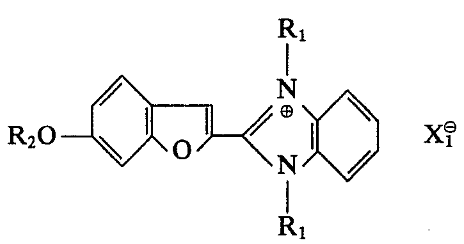

(12)

durch Umsetzung einer Verbindung der Formel

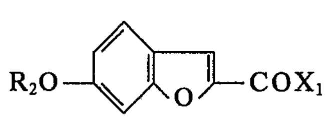

(13)

mit einer Verbindung der Formel

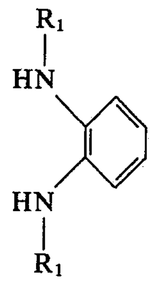

(14)

worin $X_1$ Halogen, $R_1$ und $R_2$ die oben angegebenen Bedeutungen haben, hergestellt. Bevorzugte Halogene sind Chlor und Brom.

Verbindungen der Formel (1) worin A Alkoxybenzofuranyl oder Alkenyloxybenzofuranyl bedeutet können auch dadurch hergestellt werden, indem man einen Salicylaldehyd der Formel

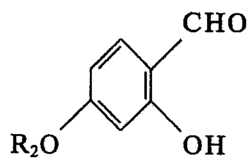

(15)

worin $R_2$ die oben angegebene Bedeutung hat, mit einem Halogenmethylbenzimidazol der Formel

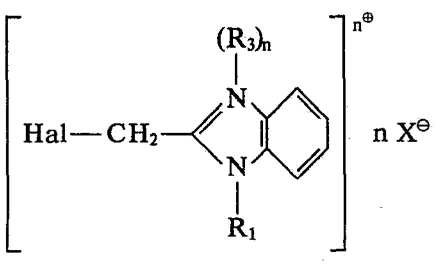

(16)

worin $R_1$, $R_3$, $X^{\ominus}$ und n die oben angegebenen Bedeutungen haben und Hal für Fluor, Chlor oder Brom steht, in An- oder Abwesenheit eines basischen Kondensationsmittels kondensiert.

Als basische Kondensationsmittel kommen anorganische und organische Verbindungen in Betracht, wie Alkalimetall- und Erdalkalimetallverbindungen, z. B. Oxide, Alkoholate, Carbonate, Bicarbonate oder Acetate, Ammoniumverbindungen, wie z. B. Ammoniumacetat oder tertiäre Amine, wie Pyridin. Vorzugsweise werden anorganische Verbindungen des Natriums und des Kaliums, besonders deren Carbonate verwendet. Es können aber auch Gemische verschiedener schwach basischer Verbindungen verwendet werden.

Die einzusetzende Menge an Kondensationsmittel bewegt sich in weiten Grenzen. Obschon für das Gelingen der Reaktion an sich eine katalytische Menge ausreichend ist, verwendet man mit Vorteil äquivalente Mengen oder sogar ein vielfaches davon.

Die Kondensation wird zweckmäßigerweise in einem unter den Reaktionsbedingungen inerten Lösungsmittel durchgeführt. Als solche Lösungsmittel kommen apolare und dipolare aprotische Lösungsmittel, wie Xylol, Dichlor- oder Trichlorbenzol, Dimethylformamid, Diäthylformamid, Dimethylacetamid, N-Methylpyrrolidon sowie deren Gemische in Betracht. Es werden vorzugsweise wasserfreie organische Lösungsmittel, worin die zur Anwendung gelangende Base teilweise oder vollständig löslich sind, verwendet.

In gewissen Fällen, z. B. wenn die Ausgangsstoffe tiefe Schmelzpunkte haben und sich nicht zersetzen, kann die erfindungsgemäße Umsetzung auch ohne Lösungsmittel, d. h. in der Schmelze, in Gegenwart des schwach basischen Kondensationsmittels durchgeführt werden.

Je nach Verfahren (mit oder ohne Lösungsmittel) und den zu kondensierenden Verbindungen kann sich die Reaktionstemperatur in einem breiten Bereich bewegen. Bei der Anwendung von Lösungsmitteln liegt sie zwischen 50° C und dem Siedepunkt des jeweiligen Lösungsmittels, vorzugsweise jedoch zwischen 50 und 200° C, besonders aber zwischen 90 und 160° C. Wird die Kondensation ohne Lösungsmittel durchgeführt, dann liegt die Reaktionstemperatur zwischen der Schmelztemperatur des Gemisches der verwendeten Reaktionskomponenten und der Zersetzungstemperatur der zu kondensierenden Verbindungen. Vorzugsweise kommen Temperaturen zwischen 100 und 250° C in Betracht.

Bevorzugte Anionen $X^{\ominus}$ sind die Halogenide wie Chloride oder Bromide sowie Tosylate. Das Anion kann gewünschtenfalls nach bekannten Methoden (vgl. z. B. Houben-Weyl, Methoden der organischen Chemie, Band XI/2, Seiten 620—626) durch ein anderes ausgetauscht werden. Bevorzugte Anionen sind in diesem Fall solche der Ameisensäure, Essigsäure, Propionsäure, Glykolsäure, Milchsäure, Äpfelsäure, Weinsäure, Schleimsäure, Gluconsäure, Citronensäure, Laevulinsäure, Acrylsäure, Methanphosphonsäure und deren Monoalkylester sowie der Phosphorsäuredialkylester.

Die Verbindungen der Formeln (10), (13), (14), (15) und (16) sind bekannt oder können nach an sich bekannten Methoden hergestellt werden (vgl. z. B. DE-OS 1 469 223, 2 031 774, 2 853 765 und 2 904 829).

Die vorstehend definierten neuen Verbindungen zeigen in gelöstem oder feinverteiltem Zustande eine mehr oder weniger ausgeprägte Fluoreszenz. Sie können zum optischen Aufhellen der verschiedensten synthetischen, halbsynthetischen oder natürlichen organischen Materialien oder Substanzen, welche solche organische Materialien enthalten, verwendet werden.

Hierfür seien beispielsweise, ohne daß durch die nachfolgende Übersicht irgendeine Beschränkung hierauf ausgedrückt werden soll, die folgenden Gruppen von organischen Materialien, soweit eine optische Aufhellung derselben in Betracht kommt, genannt:

I. Synthetische organische hochmolekulare Materialien:

a) Polymerisationsprodukte auf Basis mindestens eine polymerisierbare Kohlenstoff-Kohlenstoff-Doppelbindung enthaltender organischer Verbindung, d. h. deren Homo- oder Copolymerisate sowie deren Nachbehandlungsprodukte wie beispielsweise Vernetzungs-, Pfropfungs- oder Abbauprodukte, Polymerisat-Verschnitte oder durch Modifizierung reaktionsfähiger Gruppen erhaltene Produkte, beispielsweise Polymerisate auf Basis von $\alpha$, $\beta$-ungesättigten Carbonsäuren oder Derivaten solcher Carbonsäuren, insbesondere von Acrylverbindungen (wie z. B. Acrylestern, Acrylsäure, Acrylnitril, Acrylamiden und deren Derivaten oder deren Methacryl-Analoga), von Olefin-Kohlenwasserstoffen (wie z. B. Äthylen, Propylen, Styrole oder Diene, ferner sogenannte ABS-Polymerisate), Polymerisate auf Basis von Vinyl- und Vinyliden-Verbindungen (wie z. B. Vinylchlorid, Vinylalkohol, Vinylidenchlorid),
b) Polymerisationsprodukte, die durch Ringöffnung erhältlich sind, z. B. Polyamide vom Polycaprolactam-Typ, ferner Polymere, die sowohl über Polyaddition als auch Polykondensation erhältlich sind, wie Polyäther oder Polyacetale,
c) Polykondensationsprodukte oder Vorkondensate auf Basis bi- oder polyfunktioneller Verbindungen mit kondensationsfähigen Gruppen, deren Homo- und Mischkondensationsprodukte sowie Produkte der Nachbehandlung, wie beispielsweise Polyester, insbesondere gesättigte (z. B. Äthylenglykolterephthalsäure-Polyester) oder ungesättigte (z. B. Maleinsäure-Dialkohol-Polykondensate sowie deren Vernetzungsprodukte mit anpolymerisierbaren Vinylmonomeren), unverzweigte sowie verzweigte (auch auf Basis höherwertiger Alkohole, wie z. B. Alkydharze) Polyester, Polyamide (z. B. Hexamethylendiaminadipat), Maleinatharze, Melaminharze, deren Vorkondensate und Analoga, Polycarbonate, Silikone,
d) Polyadditionsprodukte wie Polyurethane (vernetzt und unvernetzt).

II. Halbsynthetische organische Materialien, z. B. Celluloseester verschiedener Veresterungsgrade (sogenanntes $2^1/_2$-Acetat, Triacetate) oder Celluloseäther, regenerierte Cellulose (Viskose, Kupferammoniak-Cellulose) oder deren Nachbehandlungsprodukte, Casein-Kunststoffe.

III. Natürliche organische Materialien animalischen oder vegetabilischen Ursprungs, beispielsweise auf Basis von Cellulose oder Proteinen wie Baumwolle, Wolle, Leinen, Seide, natürliche Lackharze, Stärke, Casein.

Die optisch aufzuhellenden organischen Materialien können den verschiedenartigsten Verarbeitungszuständen (Rohstoffe, Halbfabrikate oder Fertigfabrikate) angehören. Sie können andererseits in Form der verschiedenartigsten geformten Gebilde vorliegen, d. h. beispielsweise als vorwiegend dreidimensional ausgedehnte Körper wie Platten, Profile, Spritzgußformlinge, verschiedenartige Werkstücke, Schnitzel, Granulate oder Schaumstoffe, ferner als vorwiegend zweidimensional ausgebildete Körper wie Filme, Folien, Lacke, Überzüge, Imprägnierungen und Beschichtungen oder als vorwiegend eindimensional ausgebildete Körper wie Fäden, Fasern, Flocken, Drähte. Die besagten Materialien können andererseits auch in ungeformten Zuständen in den verschiedenartigsten homogenen oder inhomogenen Verteilungsformen, wie z. B. als Pulver, Lösungen, Emulsionen, Dispersionen, Latices, Pasten oder Wachse vorliegen.

Fasermaterialien können beispielsweise als endlose Fäden (verstreckt oder unverstreckt), Stapelfasern, Flocken, Strangware, textile Fäden, Garne, Zwirne, Faservliese, Filze, Watten, Beflockungs-Gebilde oder als textile Gewebe oder textile Verbundstoffe, Gewirke sowie als Papiere, Pappen oder Papiermassen vorliegen.

Den erfindungsgemäß anzuwendenden Verbindungen kommt u. a. Bedeutung für die Behandlung von textilen organischen Materialien, insbesondere textilen Geweben, zu. Sofern Fasern, welche als Stapelfasern oder Endlosfäden, in Form von Strangen, Geweben, Gewirken, Vliesen, beflockten Substraten oder Verbundstoffen vorliegen können, erfindungsgemäß optisch aufzuhellen sind, geschieht dies mit Vorteil in wäßrigem Medium, worin die betreffenden Verbindungen in feinverteilter Form (Suspensionen, sogenannten Mikrodispersionen, gegebenenfalls Lösungen) vorliegen. Gegebenenfalls können bei der Behandlung Dispergier-, Stabilisier-, Netz- und weitere Hilfsmittel zugesetzt werden.

In Abhängigkeit vom verwendeten Aufheller-Verbindungstyp kann es sich als vorteilhaft erweisen, in neutralem oder alkalischem oder saurem Bade zu arbeiten. Die Behandlung wird üblicherweise bei Temperaturen von etwa 20 bis 140° C, beispielsweise bei Siedetemperatur des Bades oder in deren Nähe (etwa 90° C), durchgeführt. Für die erfindungsgemäße Veredlung textiler Substrate kommen auch Lösungen oder Emulsionen in organischen Lösungsmitteln in Betracht, wie dies in der Färbereipraxis in der sogenannten Lösungsmittelfärberei (Foulard-Thermofixierapplikation, Ausziehfärbeverfahren in Färbemaschinen) praktiziert wird.

Die neuen optischen Aufhellmittel gemäß vorliegender Erfindung können ferner den Materialien vor oder während deren Verformung zugesetzt bzw. einverleibt werden. So kann man sie beispielsweise bei der Herstellung von Filmen, Folien (z. B. Einwalzen in Polyvinylchlorid in der Hitze) oder Formkörpern der Preßmasse oder Spritzgußmasse beifügen.

Sofern die Formgebung voll- oder halbsynthetischer organischer Materialien durch Spinnverfahren bzw. über Spinnmassen erfolgt, können die optischen Aufheller nach folgenden Verfahren appliziert werden:

— Zugabe zu den Ausgangssubstanzen (z. B. Monomeren) oder Zwischenprodukten (z. B. Vorkondensaten, Präpolymeren), d. h. vor oder während der Polymerisation, Polykondensation oder Polyaddition,
— Aufpudern auf Polymerisatschnitzel oder Granulate für Spinnmassen,
— Badfärbung von Polymerisatschnitzeln oder Granulaten für Spinnmassen,
— Dosierte Zugabe zu Spinnschmelzen oder Spinnlösungen,
— Applikation auf Spinnkabel vor dem Verstrecken.

Die neuen optischen Aufhellmittel gemäß vorliegender Erfindung können beispielsweise auch in folgenden Anwendungsformen eingesetzt werden:

a) Mischungen mit Farbstoffen (Nuancierung) oder Pigmenten (Farb- oder insbesondere z. B. Weißpigmenten) oder als Zusatz zu Färbebädern, Druck-, Ätz- oder Reservepasten. Ferner auch zur Nachbehandlung von Färbungen, Drucken oder Ätzdrucken,
b) in Mischungen mit sogenannten »Carriern«, Netzmitteln, Weichmachern, Quellmitteln, Antioxydantien, Lichtschutzmitteln, Hitzestabilisatoren, chemischen Bleichmitteln (Chlorit-Bleiche, Bleichbäder-Zusätze),
c) in Mischungen mit Vernetzern, Appreturmitteln (z. B. Stärke oder synthetischen Appreturen) sowie in Kombination mit den verschiedensten Textilveredlungsverfahren, insbesondere Kunstharzausrüstungen (z. B. Knitterfest-Ausrüstungen wie »wash-and-wear«, »permanent-press«, »no-iron«), ferner Flammfest-, Weichgriff-, Schmutzlöse (»anti-soiling«)- oder Antistatisch-Ausrüstungen oder antimikrobiellen Ausrüstungen,
d) Einarbeiten der optischen Aufhellmittel in polymere Trägermaterialien (Polymerisations-, Polykondensations- oder Polyadditionsprodukte) in gelöster oder dispergierter Form für Anwendung z. B. in Beschichtungs-, Imprägnier- oder Bindemitteln (Lösungen, Dispersionen, Emulsionen) für Textilien, Vliese, Papier, Leder,
e) als Zusätze zu sogenannten »master batches«,
f) als Zusätze zu den verschiedensten industriellen Produkten, um dieselben marktfähiger zu machen (z. B. Aspektverbesserung von Seifen, Waschmitteln, Pigmenten),
g) in Kombination mit anderen, optisch aufhellend wirkenden Substanzen,
h) in Spinnbadpräparationen, d. h. als Zusätze zu Spinnbädern, wie sie zur Gleitfähigkeitsverbesserung für die Weiterverarbeitung von Synthese-Fasern verwendet werden, oder aus einem speziellen Bad vor der Verstreckung der Faser,
i) als Szintillatoren, für verschiedene Zwecke photographischer Art, wie z. B. für elektrophotographische Reproduktion oder Supersensibilisierung,
j) je nach Substitution als Laser-Farbstoffe.

Wird das Aufhellverfahren mit Textil-Behandlungs- oder Veredlungsmethoden kombiniert, so kann die kombinierte Behandlung in vielen Fällen vorteilhafterweise mit Hilfe entsprechender beständiger Präparate erfolgen, welche die optisch aufhellenden Verbindungen in solcher Konzentration enthalten, daß der gewünschte Aufhelleffekt erreicht wird.

In gewissen Fällen werden die Aufheller durch eine Nachbehandlung zur vollen Wirkung gebracht. Diese kann beispielsweise eine chemische (z. B. Säure-Behandlung), eine thermische (z. B. Hitze) oder eine kombinierte chemisch/thermische Behandlung darstellen. So verfährt man beispielsweise bei der optischen Aufhellung einer Reihe von Fasersubstraten, z. B. von Polyesterfasern, mit den erfindungsgemäßen Aufhellern zweckmäßig in der Weise, daß man diese Fasern mit den wäßrigen Dispersionen (gegebenenfalls auch Lösungen) der Aufhellmittel bei Temperaturen unter 75° C, z. B. bei Raumtemperatur, imprägniert und einer trockenen Wärmebehandlung bei Temperaturen über 100° C unterwirft,

wobei es sich im allgemeinen empfiehlt, das Fasermaterial vorher noch bei mäßig erhöhter Temperatur, z. B. bei mindestens 60°C bis 130°C zu trocknen. Die Wärmebehandlung in trockenem Zustande erfolgt dann vorteilhaft bei Temperaturen zwischen 120 und 225°C, beispielsweise durch Erwärmen in einer Trockenkammer, durch Bügeln im angegebenen Temperaturintervall oder auch durch Behandeln mit trockenem, überhitzten Wasserdampf. Die Trocknung und trockene Wärmebehandlung können auch unmittelbar nacheinander ausgeführt oder in einen einzigen Arbeitsgang zusammengelegt werden.

Die Menge der erfindungsgemäß zu verwendenden neuen optischen Aufheller, bezogen auf das optisch aufzuhellende Material, kann in weiten Grenzen schwanken. Schon mit sehr geringen Mengen, in gewissen Fällen z. B. solchen von 0,0001 Gewichtsprozent, kann ein deutlicher und haltbarer Effekt erzielt werden. Es können aber auch Mengen bis zu etwa 0,8 Gewichtsprozent und gegebenenfalls bis zu etwa 2 Gewichtsprozent zur Anwendung gelangen. Für die meisten praktischen Belange sind vorzugsweise Mengen zwischen 0,0005 und 0,5 Gewichtsprozent von Interesse.

Aus verschiedenen Gründen ist es oft zweckmäßig, die Aufheller nicht als solche, d. h. rein einzusetzen, sondern vermischt mit den verschiedensten Hilfs- und Coupiermitteln, wie z. B. Natriumformiat, -acetat oder -lactat.

Die neuen optischen Aufheller der Formel (1) und untergeordneter Formeln worin n die Zahl 1 ist, haben den besonderen Vorteil, daß sie chloritbeständig sind.

Gegenüber nächstvergleichbaren Verbindungen gemäß DE-OS 1 469 227 und 2 159 469 zeichnen sich die erfindungsgemäßen Benzimidazolverbindungen durch eine bessere Aufhellwirkung auf Polyacrylnitrilgewebe aus.

In den Beispielen sind Teile, soweit nicht anders angegeben, immer Gewichtsteile und Prozente immer Gewichtsprozente. Schmelz- und Siedepunkte sind, sofern nicht anders vermerkt, unkorrigiert.

## Beispiel 1

Man verrührt eine Mischung von 27,3 g 2-Benzimidazolyl-(2')-6-methoxy-benzofuran (vgl. DOS 2 031 774), 100 ml Chloracetonitril und 14,7 g wasserfreies gemahlenes Kaliumcarbonat während 6 Stunden bei Rückflußtemperatur. Das Ausgangsprodukt geht dabei in Lösung, wonach sich das Reaktionsprodukt ausscheidet. Nach Abkühlen auf Raumtemperatur wird genutscht und der Rückstand wiederholt mit Isopropanol und Wasser gewaschen. Man erhält 19,3 g der Verbindung der Formel

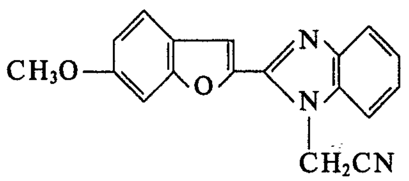

(100)

welche aus Perchloräthylen und Xylol umkristallisiert wird (hellgelbe Kristalle vom Smp. 188—91°C).

In ähnlicher Weise erhält man die Verbindungen der Formel

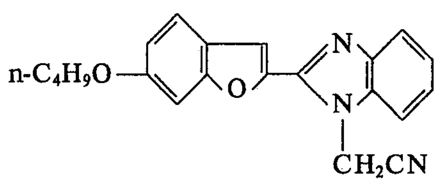

(101)

und

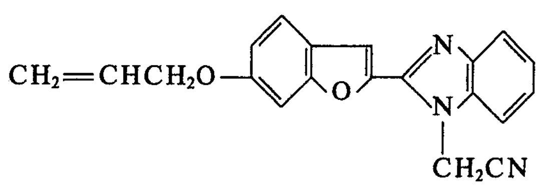

(102)

Beispiel 2

Verwendet man in Beispiel 1 anstelle von Chloracetonitril 60 ml 2-Brompropionitril und fügt im Verlauf der Reaktion noch weitere 8,9 g Kaliumcarbonat portionenweise hinzu, so erhält man die Verbindung der Formel

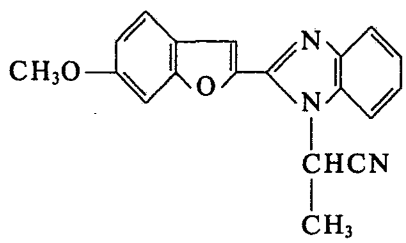

(200)

Die Reinigung erfolgt durch chromatographieren an Aluminiumoxid neutral Aktivität 1 mit Äthylenchlorid und anschließender Umkristallisation aus Isopropanol (Smp. 72° C).

Beispiel 3

Zu einer heißen Lösung von 27,3 g 2-Benzimidazolyl-(2')-6-methoxy-benzofuran in 450 ml Methanol tropft man unter Rühren 19,8 g einer 30%igen Natriummethylat-Lösung. Man destilliert das Methanol vollständig ab, zuletzt im Vakuum. Zum festen Rückstand fügt man 50 ml Bromessigsäureäthylester hinzu, verrührt die Suspension 1 Stunde bei 50° C, verdünnt nach dem Abkühlen auf Raumtemperatur mit 250 ml Methylenchlorid und filtriert von unlöslichem Material. Die erhaltene Lösung wird im Vakuum eingedampft und der Rückstand in 450 ml heißem Äthylenchlorid aufgenommen. Beim Abkühlen auf 0° C fällt das Produkt dick aus. Nach Absaugen, Waschen mit Äthylenchlorid und Trocknen im Vakuum bei 70° C erhält man 33,5 g der Verbindung der Formel

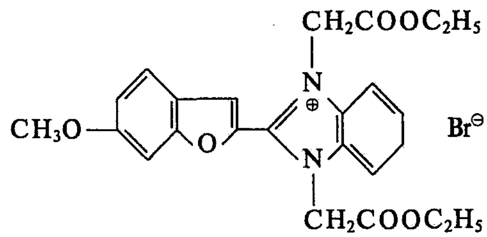

(300)

die aus Isopropanol umkristallisiert, in Form von annähernd farblosen Kristallen vom Smp. 178—180° C (Zers.) anfällt.

Beispiel 4

Zu einer Suspension von 27,3 g 2-Benzimidazolyl-(2')-6-methoxy-benzofuran in 100 ml Äthanol gibt man unter Rühren bei 70° C 11 ml einer 10 n wässerigen Kaliumhydroxidlösung und dampft die entstandene Lösung im Vakuum vollständig ein. Zum festen Rückstand fügt man 50 ml Chloressigsäureäthylester hinzu, verrührt die Suspension 1/2 Stunde bei 130° C, verdünnt nach dem Abkühlen mit 100 ml Methylenchlorid und filtriert von unlöslichem Material. Die Lösung wird im Vakuum eingedampft und der Rückstand in 100 ml warmem Aceton aufgenommen. Beim Abkühlen auf 0° C fällt das Produkt langsam aus. Nach Absaugen, Waschen mit Aceton und Trocknen im Vakuum bei Raumtemperatur erhält man 10,2 g der Verbindung der Formel

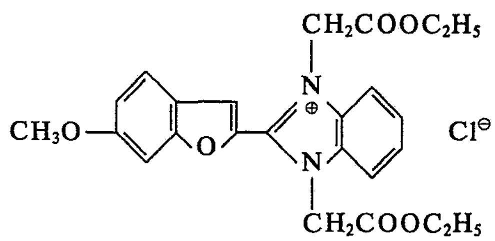

(400)

Diese kann aus Aceton umkristallisiert werden: Smp. 153—158° C (Zers.).

Beispiel 5

Man verrührt eine Mischung von 27,3 g 2-Benzimidazolyl-(2')-6-methoxy-benzofuran, 80 ml Chloressigsäuremethylester und 15,2 g wasserfreies, gemahlenes Kaliumcarbonat während 4 Stunden bei 90° C und filtriert heiß von unlöslichem Material. Das Filtrat wird im Vakuum vollständig eingedampft und in 80 ml warmem Methanol aufgenommen. Nach dem Abkühlen auf 0° C fügt man zur Lösung unter Rühren 50 ml Wasser hinzu, wobei das Reaktionsprodukt allmählich ausfällt. Dieses wird abgenutscht, wiederholt mit einer Mischung von Methanol-Wasser 1 : 1 gewaschen und im Vakuum über Calciumchlorid getrocknet. Nach Umkristallisieren aus Tetrachlorkohlenstoff erhält man 23,0 g der Verbindung der Formel

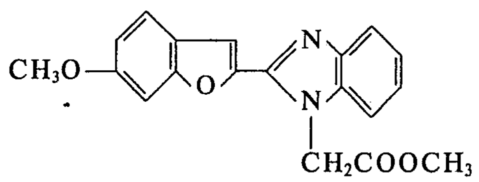

(500)

Smp. 111—113° C (aus Isopropanol).

8,4 g dieses Produktes werden in 20 ml Chloracetonitril 2 Stunden bei 120° C verrührt und die Lösung am Rotationsverdampfer im Vakuum vollständig eingedampft. Der Rückstand wird in 70 ml Chloroform aufgenommen, das ausgefallene Produkt abgenutscht, dreimal mit je 20 ml Chloroform gewaschen und im Vakuum getrocknet. Man erhält 7,7 g der Verbindung der Formel

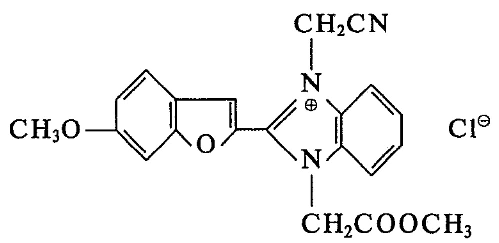

(501)

vom Smp. 150° C (Zers.). Die Verbindung läßt sich aus Äthanol umkristallisieren.

Beispiel 6

Eine Suspension von 4,2 g 6-Methoxy-cumarilsäurechlorid und 4,3 g der Verbindung der Formel

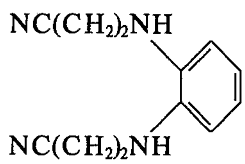

(600)

in 20 ml Pyridin wird 1/2 Stunde bei 100° C verrührt. Beim Abkühlen der Lösung auf 5° C fällt das Reaktionsprodukt aus. Es wird abgenutscht, wiederholt mit Aceton und Toluol gewaschen und im Vakuum bei 100° C getrocknet. Man erhält 5,9 g der Verbindung der Formel

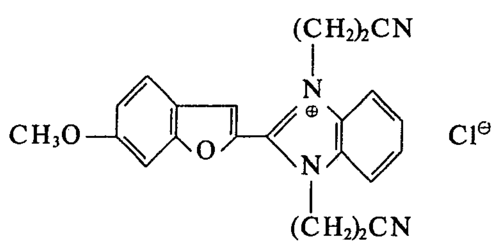

(601)

in Form hellgelber Kristalle vom Smp. 215—216° C (Zers.).

Beispiel 7

39,6 g 2-Benzimidazolyl-(2')-6-methoxy-benzofuran werden in 180 ml Acrylnitril und 18,5 g Triäthylamin 44 Stunden bei Rückflußtemperatur verrührt. Man saugt das Triäthylamin und das überschüssige Acrylnitril ab, löst den Rückstand in Toluol, filtriert heiß und läßt abkühlen. Das ausgefallene Produkt wird abgenutscht, mit Toluol gewaschen und im Vakuum bei 100° C getrocknet. Man erhält 43,2 g der Verbindung der Formel

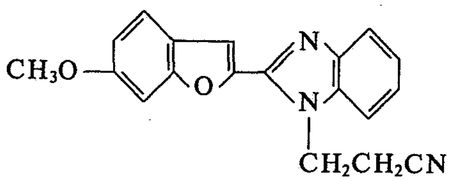

(700)

Smp. 131—133° C (nach Umkristallisation aus Toluol).

Beispiel 8

29,5 g 2-Benzimidazolyl-(2')-5-(p-chlorphenyl)-furan werden in 20 ml Methanol und 11 ml 10-n Natronlauge in der Wärme gelöst. Man dampft die Lösung im Vakuum vollständig ein, fügt 100 ml Chloracetonitril hinzu und verrührt die Suspension 4 Stunden bei 70° C. Nach dem Abkühlen auf Raumtemperatur verdünnt man mit 100 ml Methanol und 10 ml Wasser, filtriert, und wäscht den Rückstand wiederholt mit Methanol und Wasser. Dieser wird im Vakuum bei 100° C getrocknet (28,0 g) und aus n-Butanol umkristallisiert. Man erhält 20,9 g der Verbindung der Formel

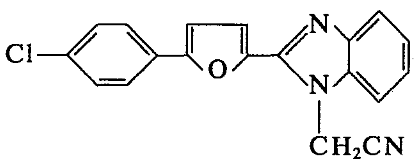

(800)

in Form blaß-gelber Kristalle, Smp. 223—226° C (aus Xylol).

6,7 g dieses Produktes werden in 20 ml Bromessigsäureäthylester 2 Stunden bei 100° C verrührt. Nachdem dieses in Lösung gegangen ist, scheidet sich das Endprodukt voluminös aus. Man verdünnt mit 50 ml Methyläthylketon, nutscht bei ca. 40° C und wäscht den Rückstand mit Methyläthylketon. Nach dem Trocknen erhält man 7,3 g der Verbindung der Formel

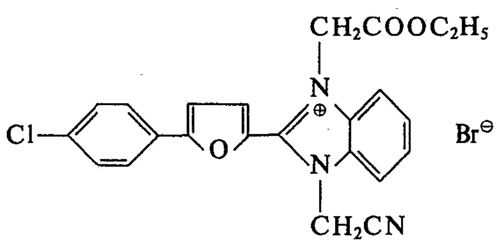

(801)

die nach Umkristallisation aus Wasser in Form hellgelber Kristalle vom Smp. 170° C (unscharf) erhalten wird.

Beispiel 9

5,9 g 2-Benzimidazolyl-(2')-5-(p-chlorphenyl)-furan und 3,3 g wasserfreies, gemahlenes Kaliumcarbonat in 30 ml Chloracetonitril 18 Stunden bei Rückflußtemperatur verrührt. Man destilliert das überschüssige Chloracetonitril im Vakuum ab, kocht den Rückstand mit 80 ml und dann 20 ml Wasser aus und dekantiert die wässerigen Phasen bei 100° C ab. Die vereinigten wässerigen Phasen werden nach Zugabe von Aktivkohle heiß klärfiltriert und etwas eingeengt, wonach das Produkt beim Abkühlen auskristallisiert. Dieses wird abgenutscht, mit Wasser gewaschen und im Vakuum bei 100° C getrocknet. Man erhält 1,8 g der Verbindung der Formel

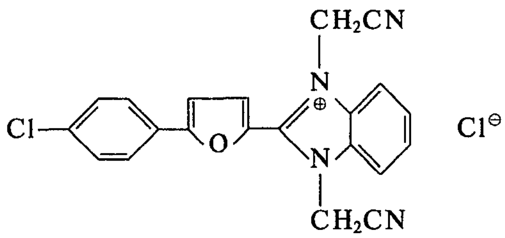

(900)

in Form hellgelber Kristalle, die noch 1/2 Mol Kristallwasser enthalten. Smp. 205—207° C (nach Umkristallisation aus Alkohol-Acetonitril 3 : 7).

Verwendet man in diesem Beispiel bei sonst gleichen Vorgehen anstelle des erwähnten Furan-derivaten eine entsprechende Menge 2-Benzimidazolyl-(2')-5-phenylfuran, so erhält man die Verbindung der Formel

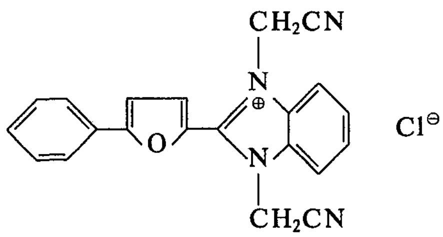

(901)

In analoger Weise erhält man die Verbindung der Formel

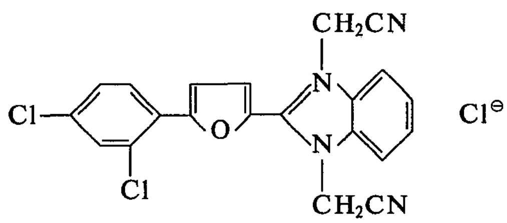

(902)

Beispiel 10

Verwendet man in Beispiel 3 als Ausgangsmaterial 2-Benzimidazolyl-(2')-5-(p-chlorphenyl)-furan anstelle des erwähnten 2-Benzimidazolyl-(2')-6-methoxy-benzofurans, so erhält man die Verbindung der Formel

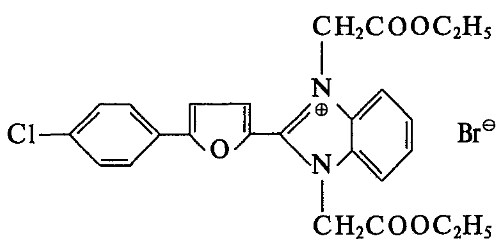

(1000)

Beispiel 11

Ein Polyacrylnitril-Gewebe (Orlon 75) auf einem Färbeapparat bei einem Flottenverhältnis von 1 : 20 mit einem wäßrigen Bad, das 0,1% des Aufhellers der Formel (100), (200), (300), (601), (801) oder (900) bezogen auf das Warengewicht, 1 g/l eines Anlagerungsproduktes von 35 Mol Äthylenoxid an 1 Mol Stearylalkohol und 1,5 ml/l Ameisensäure 85% enthält, behandelt. Die Applikation erfolgt gemäß folgendem Temperaturprogramm: 40—97° C/30 Minuten, 97° C/30 Minuten, 97—40° C/15 Minuten. Anschließend wird das Polyacrylnitril-Gewebe während 30 Sekunden in fließendem enthärtetem Wasser gespült und bei 70° C im Trockenschrank getrocknet. Das so behandelte Gewebe weist einen hohen Aufhelleffekt auf.

Beispiel 12

Ein modifiziertes Polyacrylnitrilgewebe (Courtelle) wird auf einem Färbeapparat bei einem Flottenverhältnis von 1 : 20 mit einem wässerigen Bad, das 0,1% des Aufhellers der Formel (300), (601) oder (900) bezogen auf das Warengewicht, 1 g/l Oxalsäure, 0,25 g/l eines Polyphosphates als Komplexbildner und 0,125 g/l Natriummetabisulfit enthält, behandelt. Die Applikation erfolgt gemäß folgendem Temperaturprogramm: 40—97° C/30 Minuten, 97° C/30 Minuten, 97—40° C/15 Minuten.

Anschließend wird das Polyacrylnitril-Gewebe während 30 Sekunden in fließendem enthärtetem Wasser gespült und bei 70° C im Trockenschrank getrocknet. Das so behandelte Gewebe weist einen guten Aufhelleffekt auf.

Beispiel 13

Frisch ausgesponnenes, verstrecktes Polyacrylnitril-Naßkabel (entsprechend 3,0 g Trockengewicht) wird noch feucht bei 45° C für 4 Sekunden in 100 ml einer wäßrigen Flotte getaucht, die 0,0005% des Aufhellers der Formel (300), (601) oder (900) enthält und mit konzentrierter Oxalsäurelösung auf pH 4 eingestellt worden ist. Anschließend spült man das Naßkabel kurz mit Wasser und trocknet bei 90 bis 100° C. Man erhält auf diese Weise eine gut aufgehellte Polyacrylnitrilfaser.

Die Ausfärbung kann z. B. auch bei pH 6 (eingestellt durch Zugabe von Natriumacetat) erfolgen. Erhöhte Temperatur der Färbeflotte, z. B. auf 40° C, erhöht die Ausziehgeschwindigkeit.

Höhere Weißeffekte werden durch Erhöhen der Aufhellerkonzentration, z. B. auf 0,005%, erzielt.

Beispiel 14

Es wird eine wäßrige Lösung hergestellt, die, bezogen auf das Gewicht des aufzuhellenden Materials, 0,3% des Aufhellers der Formel (300), (801) oder (900) enthält. Diese Lösung wird auf 30° C erwärmt.

Dann gibt man ein durch Cokondensation mit 2 bis 5 Mol.-% Isophthalsäure-5-Natriumsulfonat hergestelltes modifiziertes Polyestergewebe (®Dacron 64) in die Lösung, wobei ein Flottenverhältnis von 1 : 25 eingehalten wird. Man steigert die Temperatur innerhalb von 10 Minuten auf 100°C und beläßt 20 Minuten bei dieser Temperatur. Dann kühlt man innerhalb 5 Minuten auf 50°C ab. Das Gewebe wird sodann in fließendem kaltem Wasser gespült und anschließend bei 180°C mit dem Bügeleisen getrocknet. Es weist einen hohen Aufhelleffekt auf.

**Patentansprüche**

1. Benzimidazole der Formel

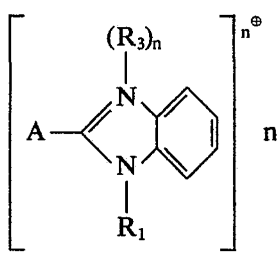 $n X^{\ominus}$ (1)

worin

A den Rest

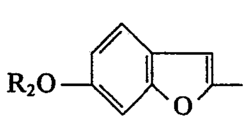 oder 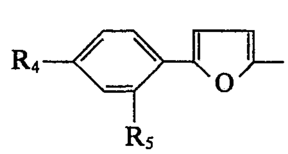

$R_1$ Cyanomethyl, 1-Cyanoäthyl oder, wenn n die Zahl 1 ist, auch 2-Cyanoäthyl oder $C_{3-6}$-Carbalkoxymethyl,

$R_2$ $C_{1-4}$-Alkyl oder $C_{3-4}$-Alkenyl,

$R_3$ Cyanomethyl, Cyanoäthyl oder $C_{3-6}$-Carbalkoxymethyl, wobei wenn $R_3$ Cyanoäthyl bedeutet, $R_1$ ist 1-Cyanoäthyl oder 2-Cyanoäthyl

$R_4$ und $R_5$ unabhängig voneinander Wasserstoff oder Chlor,
$X^{\ominus}$ ein farbloses Anion einer organischen oder anorganischen Säure und
n die Zahl 0 oder 1

bedeuten.

2. Benzimidazole gemäß Anspruch 1 der Formel

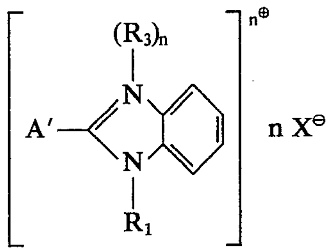

(2)

worin

A' den Rest

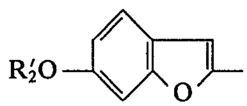

oder

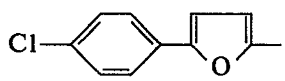

$R_1$ Cyanomethyl, 1-Cyanoäthyl oder, wenn n die Zahl 1 ist, auch 2-Cyanoäthyl oder $C_{3-6}$-Carbalkoxymethyl,
$R'_2$ $C_{1-4}$-Alkyl,
$R_3$ Cyanomethyl, Cyanoäthyl oder $C_{3-6}$-Carbalkoxymethyl, wobei wenn $R_3$ Cyanoäthyl bedeutet, $R_1$ ist 1-Cyanoäthyl oder 2-Cyanoäthyl,
$X^{\ominus}$ ein farbloses Anion einer organischen oder anorganischen Säure und
n die Zahl 0 oder 1

bedeuten.

3. Benzimidazole gemäß Anspruch 1 der Formel

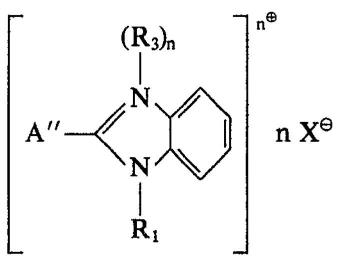

(3)

worin

A'' den Rest

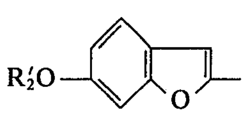

oder, wenn n die Zahl 1 ist, auch den Rest

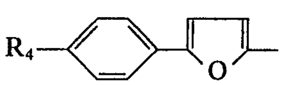

$R_1$ Cyanomethyl, 1-Cyanoäthyl oder wenn n die Zahl 1 ist auch 2-Cyanoäthyl oder $C_{3-6}$-Carbalkoxymethyl,
$R'_2$ $C_{1-4}$-Alkyl,
$R_3$ Cyanomethyl, Cyanoäthyl oder $C_{3-6}$-Carbalkoxymethyl, wobei wenn $R_3$ Cyanoäthyl bedeutet, $R_1$ ist 1-Cyanoäthyl oder 2-Cyanoäthyl,

$X^{\ominus}$ ein farbloses Anion einer organischen oder anorganischen Säure und
n die Zahl 0 oder 1

bedeuten.

4. Benzimidazole gemäß Anspruch 2 der Formel

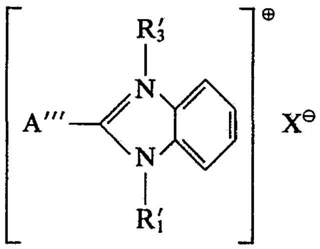

(4)

worin

A''' den Rest

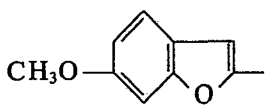

oder

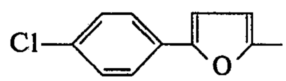

$R'_1$ Cyanomethyl, Cyanoäthyl oder $C_{3-6}$-Carbalkoxymethyl,
$R'_3$ Cyanomethyl, Cyanoäthyl oder $C_{3-6}$-Carbalkoxymethyl, wobei wenn $R'_3$ Cyanoäthyl bedeutet, $R'_1$ ist Cyanoäthyl und
$X^{\ominus}$ ein farbloses Anion einer organischen oder anorganischen Säure

bedeuten.

5. Benzimidazole gemäß Anspruch 4 der Formel

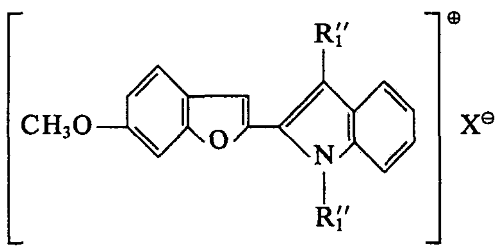

(5)

worin

$R''_1$ Cyanoäthyl oder $C_{3-4}$-Carbalkoxymethyl und
$X^{\ominus}$ ein farbloses Anion einer anorganischen oder organischen Säure

bedeuten.

6. Benzimidazole gemäß Anspruch 4 der Formel

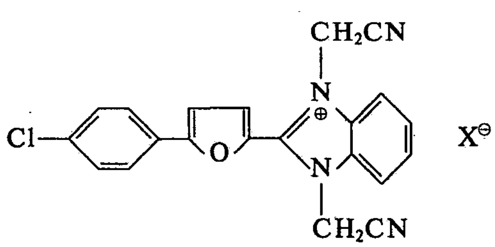

(6)

worin $X^{\ominus}$ ein farbloses Anion einer organischen oder anorganischen Säure bedeutet.

7. Das Benzimidazol gemäß Anspruch 3 der Formel

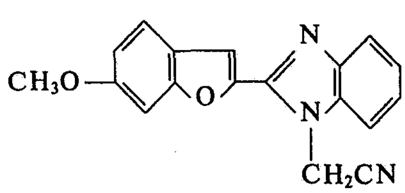

8. Benzimidazole gemäß Anspruch 5 der Formel

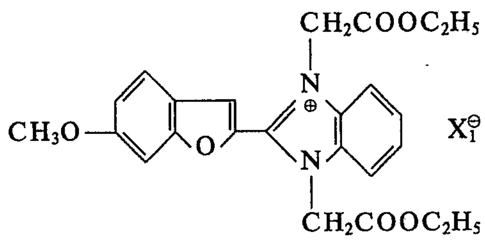

worin $X_1$ Chlor oder Brom bedeutet.

9. Das Benzimidazol gemäß Anspruch 5 der Formel

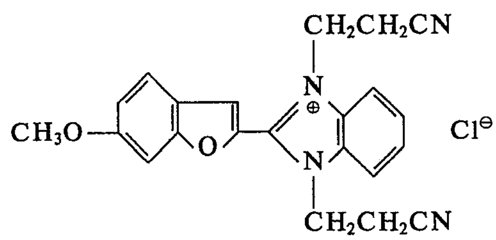

10. Verfahren zur Herstellung von Benzimidazolen der Formel

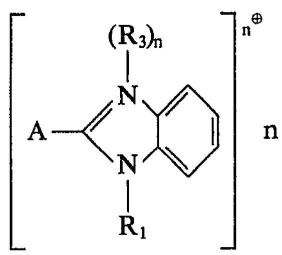

worin

| | |
|---|---|
| A | den Rest |

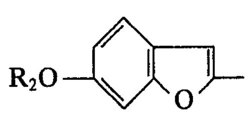

oder

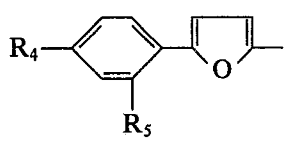

| | |
|---|---|
| $R_1$ | Cyanomethyl, 1-Cyanoäthyl oder, wenn n die Zahl 1 ist, auch 2-Cyanoäthyl oder $C_{3-6}$-Carbalkoxymethyl, |
| $R_2$ | $C_{1-4}$-Alkyl oder $C_{3-4}$-Alkenyl, |
| $R_3$ | Cyanomethyl, Cyanoäthyl oder $C_{3-6}$-Carbalkoxymethyl, wobei wenn $R_3$ Cyanoäthyl bedeutet, $R_1$ ist 1-Cyanoäthyl oder 2-Cyanoäthyl, |
| $R_4$ und $R_5$ | unabhängig voneinander Wasserstoff oder Chlor, |
| $X^{\ominus}$ | ein farbloses Anion einer organischen oder anorganischen Säure und |
| n | die Zahl 0 oder 1 |

bedeuten, dadurch gekennzeichnet, daß man ein Benzimidazol der Formel

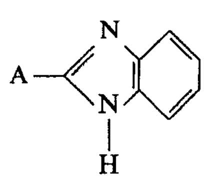

(10)

in einer ersten Stufe in Gegenwart einer Base mit mindestens einem Moläquivalent eines Alkylierungsmittels $R_1X$ oder mit Acrylnitril in ein Benzimidazol der Formel

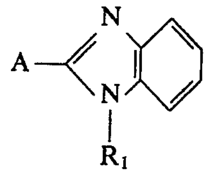

(11)

überführt und dieses gewünschtenfalls in einer zweiten Phase, ohne oder unter vorausgehender Isolierung, mit einer Verbindung der Formel $R_3X$ quaterniert, wobei in den obigen Formeln A, $R_1$, $R_3$ und X die oben angegebenen Bedeutungen haben.

11. Verfahren gemäß Anspruch 10, worin die Alkylierungs- und Quaternierungsreaktion in überschüssigem Alkylierungsmittel oder in einem inerten organischen Lösungsmittel bei Temperaturen zwischen 20 und 150° C vorgenommen wird.

12. Verfahren zur Herstellung von Benzimidazolen der Formel

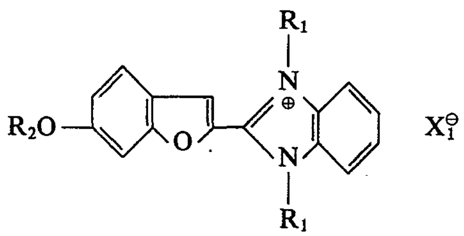

worin

$R_1$ Cyanomethyl, 1-Cyanoäthyl, 2-Cyanoäthyl oder $C_{3-4}$-Carbalkoxymethyl,
$R_2$ $C_{1-4}$-Alkyl oder $C_{3-4}$-Alkenyl und
$X_1$ Halogen

bedeuten, dadurch gekennzeichnet, daß man, in einem inerten Lösungsmittel und bei Temperaturen zwischen 20 und 150° C ein Cumarilsäurehalogenid der Formel

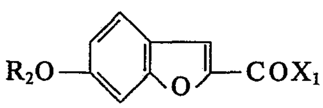

mit einem o-Phenylendiamin der Formel

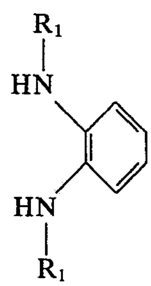

in welchen Formeln $R_1$, $R_2$ und $X_1$ die oben angegebene Bedeutung haben, umsetzt.

13. Verfahren zur Herstellung von Benzofuranyl-benzimidazolen der Formel

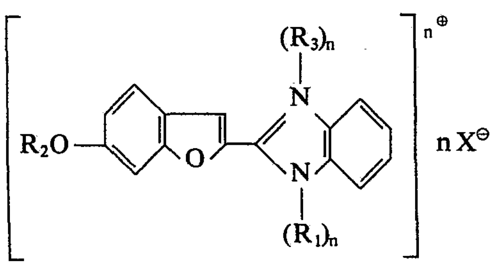

$R_1$ Cyanomethyl, 1-Cyanoäthyl oder, wenn n die Zahl 1 ist, auch 2-Cyanoäthyl oder $C_{3-6}$-Carbalkoxymethyl,

$R_2$ $C_{1-4}$-Alkyl oder $C_{3-4}$-Alkenyl,
$R_3$ Cyanomethyl, Cyanoäthyl oder $C_{3-6}$-Carbalkoxymethyl, wobei wenn $R_3$ Cyanoäthyl bedeutet, $R_1$ ist 1-Cyanoäthyl oder 2-Cyanoäthyl
$X^{\ominus}$ ein farbloses Anion einer anorganischen oder organischen Säure und
n die Zahl 0 oder 1

bedeuten, dadurch gekennzeichnet, daß man einen Salicylaldehyd der Formel

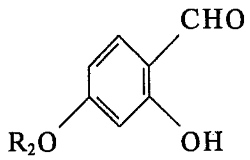

worin $R_2$ die oben angegebene Bedeutung hat, mit einem Halogenmethylbenzimidazol der Formel

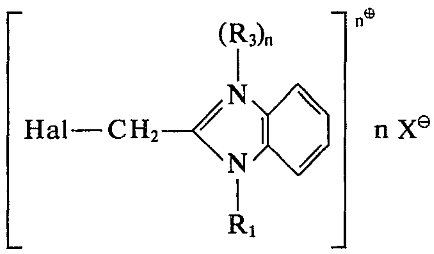

worin $R_1$, $R_3$, $X^{\ominus}$ und n die oben angegebenen Bedeutungen haben und Hal für Fluor, Chlor oder Brom steht, in An- oder Abwesenheit eines basischen Kondensationsmittels, kondensiert.

14. Verfahren zum optischen Aufhellen von organischen Materialien, dadurch gekennzeichnet, daß man den aufzuhellenden Materialien, Benzimidazole der in den Ansprüchen 1 bis 9 definierten Formeln einverleibt oder auf besagte Materialien aufbringt.

15. Verfahren gemäß Anspruch 14, dadurch gekennzeichnet, daß man als organisches Material Polyacrylnitril verwendet.

16. Verfahren gemäß Anspruch 14, dadurch gekennzeichnet, daß man 0,001 bis 2% des Aufhellers, bezogen auf das Gewicht des aufzuhellenden Materials, verwendet.

17. Verwendung der in den Ansprüchen 1 bis 9 definierten Benzimidazole als optische Aufhellmittel für organische Materialien.

18. Verwendung gemäß Anspruch 17 der in den Ansprüchen 1 bis 9 definierten Benzimidazole als optische Aufhellmittel für Polyacrylnitril.

19. Verwendung gemäß Anspruch 17 der in den Ansprüchen 1 bis 5, 8 und 9 definierten quaternierten Benzimidazole als optische Aufhellmittel für mit sauren Gruppen modifizierten Polyester.

20. Organisches Material, enthältend 0,001 bis 2 Gewichtsprozent von in Anspruch 1 definierten Benzimidazolen.

## Claims

1. A benzimidazole of the formula

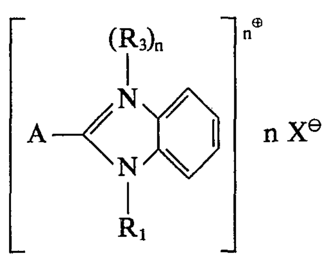

(1)

in which

A is the radical

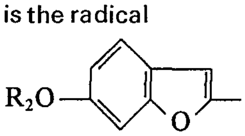

or

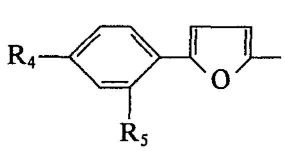

$R_1$ is cyanomethyl, 1-cyanoethyl or, if n is the number 1, also 2-cyanoethyl or $C_{3-6}$-carboalkoxymethyl,
$R_2$ is $C_{1-4}$-alkyl or $C_{3-4}$-alkenyl,
$R_3$ is cyanomethyl, cyanoethyl or $C_{3-6}$-carboalkoxymethyl, $R_1$ being 1-cyanoethyl or 2-cyanoethyl when $R_3$ is cyanoethyl,
$R_4$ and $R_5$ independently of one another are hydrogen or chlorine,
$X^{\ominus}$ is a colourless anion of an organic or inorganic acid and
n is the number 0 or 1.

2. A benzimidazole according to claim 1 of the formula

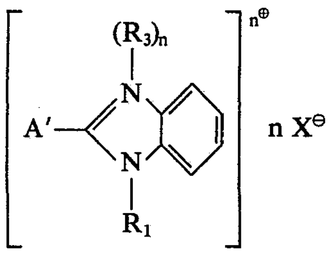

(2)

in which

A' is the radical

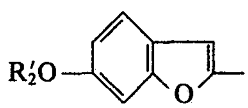

or

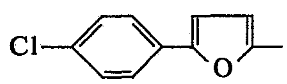

$R_1$ is cyanomethyl, 1-cyanoethyl or, if n is the number 1, also 2-cyanoethyl or $C_{3-6}$-carboalkoxymethyl,
$R'_2$ is $C_{1-4}$-alkyl,
$R_3$ is cyanomethyl, cyanoethyl or $C_{3-6}$-carboalkoxymethyl, $R_1$ being 1-cyanoethyl or 2-cyanoethyl when $R_3$ is cyanoethyl,
$X^{\ominus}$ is a colourless anion of an organic or inorganic acid and
n is the number 0 or 1.

3. A benzimidazole according to claim 1 of the formula

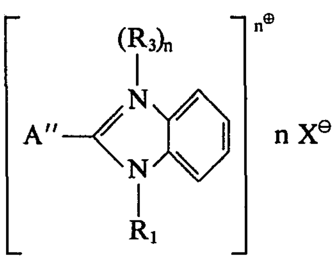

(3)

in which

A'' is the radical

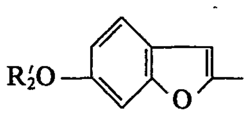

or, if n is the number 1, also the radical

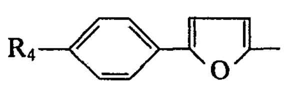

$R_1$ is cyanomethyl, 1-cyanoethyl or, if n is the number 1, also 2-cyanoethyl or $C_{3-6}$-carboalkoxymethyl,
$R'_2$ is $C_{1-4}$-alkyl,
$R_3$ is cyanomethyl, cyanoethyl or $C_{3-6}$-carboalkoxymethyl, $R_1$ being 1-cyanoethyl or 2-cyanoethyl when $R_3$ is cyanoethyl,
$X^\ominus$ is a colourless anion of an organic or inorganic acid and
n is the number 0 or 1.

4. A benzimidazole according to claim 2 of the formula

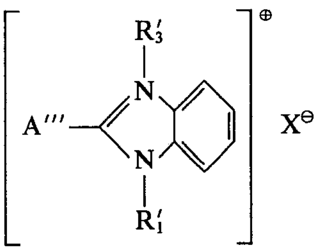

(4)

in which

A''' is the radical

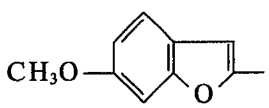

or

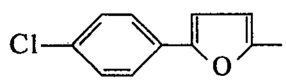

$R'_1$ is cyanomethyl, cyanoethyl or $C_{3-6}$-carbalkoxymethyl,
$R'_3$ is cyanomethyl, cyanoethyl or $C_{3-6}$-carbalkoxymethyl,
$R'_1$ being cyanoethyl when $R'_3$ is cyanoethyl, and
$X^\ominus$ is a colourless anion of an organic or inorganic acid.

5. A benzimidazole according to claim 4 of the formula

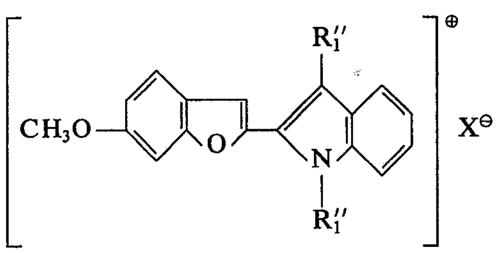

(5)

in which

$R''_1$ is cyanoethyl or $C_{3-4}$-carboalkoxymethyl and
$X^\ominus$ is a colourless anion of an inorganic or organic acid.

6. A benzimidazole according to claim 4 of the formula

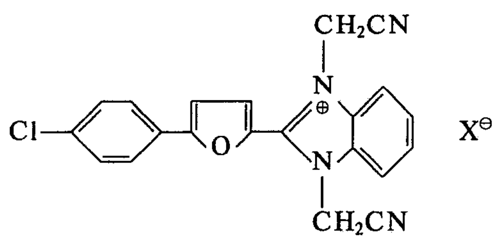

(6)

in which $X^\ominus$ is a colourless anion of an organic or inorganic acid.

7. The benzimidazole according to claim 3 of the formula

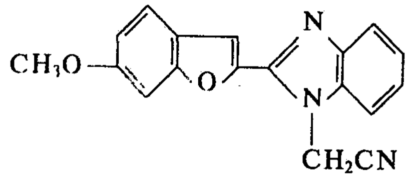

8. A benzimidazole according to claim 5 of the formula

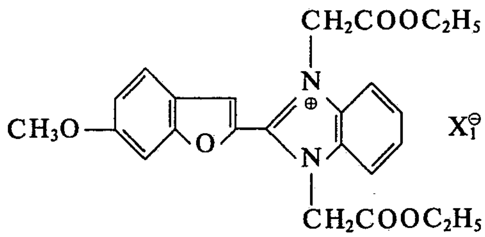

in which $X_1$ is chlorine or bromine.

9. The benzimidazole according to claim 5 of the formula

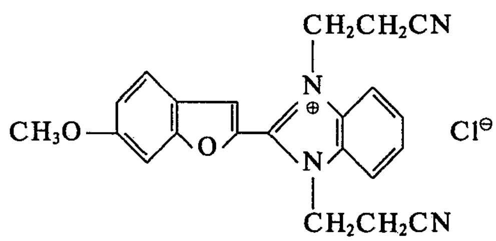

10. A process for the preparation of a benzimidazole of the formula

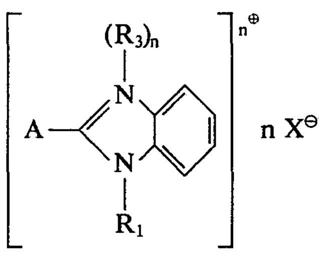

in which

| | |
|---|---|
| A | is the radical |

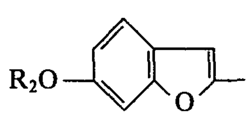

or

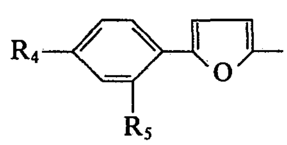

| | |
|---|---|
| $R_1$ | is cyanomethyl, 1-cyanoethyl or, if n is the number 1, also 2-cyanoethyl or $C_{3-6}$-carboalkoxymethyl, |
| $R_2$ | is $C_{1-4}$-alkyl or $C_{3-4}$-alkenyl, |
| $R_3$ | is cyanomethyl, cyanoethyl or $C_{3-6}$-carboalkoxymethyl, $R_1$ being 1-cyanoethyl or 2-cyanoethyl when $R_3$ is cyanoethyl, |
| $R_4$ and $R_5$ | independently of one another are hydrogen or chlorine, |
| $X^{\ominus}$ | is a colourless anion of an organic or inorganic acid and |
| n | is the number 0 or 1, |

which comprises converting a benzimidazole of the formula

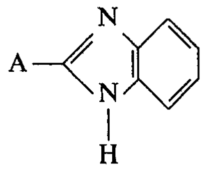

(10)

in a first stage, in the presence of a base, by means of at least one mol equivalent of an alkylating agent $R_1X$ or by means of acrylonitrile, into a benzimidazole of the formula

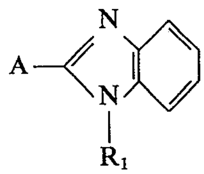

(11)

and, if desired, with or without previously isolating the latter, quaternising it in a second phase with a compound of the formula $R_3X$, A, $R_1$, $R_3$ and X in the above formulae being as defined above.

11. A process according to claim 10, wherein the alkylation and quaternisation reaction is carried out at temperatures between 20 and 150° C in excess alkylating agent or in an inert organic solvent.

12. A process for the preparation of a benzimidazole of the formula

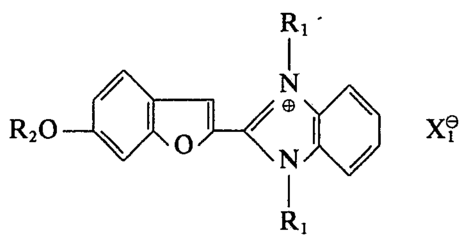

in which

$R_1$ is cyanomethyl, 1-cyanoethyl, 2-cyanoethyl or $C_{3-4}$-carbalkoxymethyl,
$R_2$ is $C_{1-4}$-alkyl or $C_{3-4}$-alkenyl and
$X_1$ is halogen,

which comprises reacting, in an inert solvent and at temperatures between 20 and 150° C, a coumarilyl halide of the formula

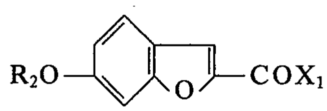

with an o-phenylenediamine of the formula

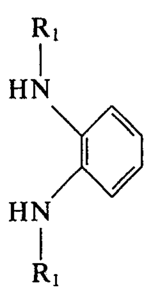

in which formulae $R_1$, $R_2$ and $X_1$ are as defined above.

13. A process for the preparation of a benzofuranyl-benzimidazole of the formula

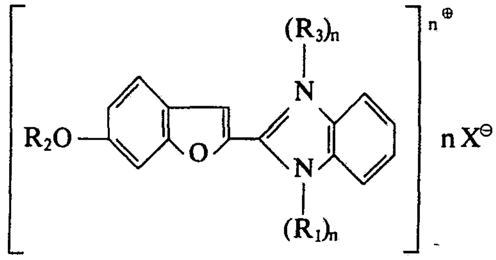

in which

$R_1$ is cyanomethyl, 1-cyanoethyl or, if n is the number 1, also 2-cyanoethyl or $C_{3-6}$-carbalkoxymethyl,
$R_2$ is $C_{1-4}$-alkyl or $C_{3-4}$-alkenyl,
$R_3$ is cyanomethyl, cyanoethyl or $C_{3-6}$-carbalkoxymethyl, $R_1$ being 1-cyanoethyl or 2-cyanoethyl when $R_3$ is cyanoethyl,
$X^{\ominus}$ is a colourless anion of an inorganic or organic acid and
n is the number 0 or 1,

which comprises subjecting a salicylaldehyde of the formula

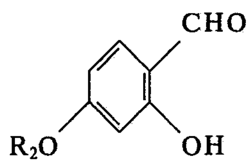

in which $R_2$ is as defined above, to a condensation reaction, in the presence or absence of a basic condensation agent, with a halogenomethylbenzimidazole of the formula

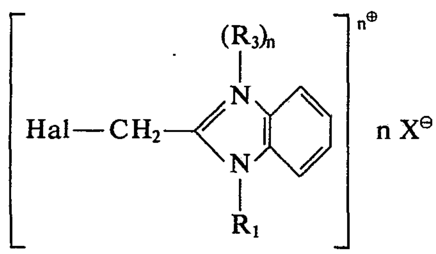

in which $R_1$, $R_3$, $X^{\ominus}$ and n are as defined above and Hal is fluorine, chlorine or bromine.

14. A process for the fluorescent brightening of organic materials, which comprises incorporating a benzimidazole of the formulae defined in claims 1 to 9 into the materials to be whitened, or applying it to the said materials.

15. A process according to claim 14, wherein polyacrylonitrile is used as the organic material.

16. A process according to claim 14, wherein 0,001 to 2% of the brightener, relative to the weight of the material to be whitened, is used.

17. Use of a benzimidazole defined in claims 1 to 9 as a fluorescent brightening agent for organic materials.

18. Use in accordance with claim 17 of a benzimidazole defined in claims 1 to 9 as a fluorescent brightening agent for polyacrylonitrile.

19. Use in accordance with claim 17 of a quaternised benzimidazole defined in claims 1 to 5, 8 and 9, as a fluorescent brightening agent for polyesters which have been modified with acid groups.

20. Organic material containing 0,001 to 2 per cent by weight of a benzimidazole defined in claim 1.

**Revendications**

1. Benzimidazoles de formule

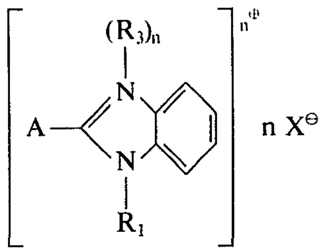

(1)

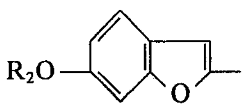

ou

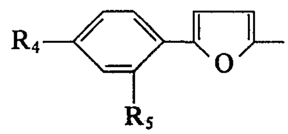

dans laquelle:

$R_1$ désigne un groupe cyanométhyle, 1-cyanoéthyle ou lorsque n est le nombre 1, également 2-cyanoéthyle ou $C_{3-6}$-carbakoxyméthyle,

$R_2$ désigne un groupe $C$-$_{1-4}$ alkyle ou $C_{3-4}$ Alcényle,

$R_3$ désigne un groupe cyanométhyle, cyanoéthyle ou $C_{3-6}$ carbalkoxyléthyle, lorsque $R_3$ désigne un groupe cyanoéthyle, $R_1$ étant un groupe 1-cyanoéthyle ou 2-cyanoéthyle.

$R_4$ et $R_5$ désignent indépendamment l'un de l'autre un atome d'hydrogène ou de chlore,

$X^{\ominus}$ désigne un anion incolore d'un acide organique ou minéral et

n 0 ou 1.

2. Benzimidazoles selon la revendication 1, de formule

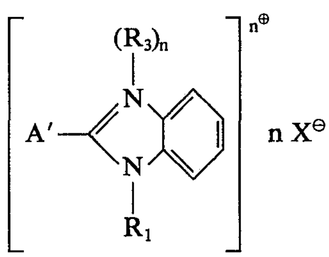

(2)

dans laquelle

A' désigne le reste

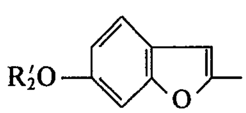

ou

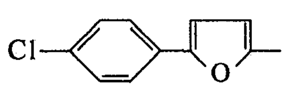

$R_1$ désigne un groupe cyanométhyle, 1-cyanoéthyle, ou lorsque n est le nombre 1, également 2-cyanoéthyle ou $C_{3-6}$ carbalkoxyméthyle

$R'_2$ désigne un groupe $C_{1-4}$ alkyle

$R_3$ désigne un groupe cyanométhyle, cyanoéthyle ou $C_{3-6}$ carbalkoxyléthyle, lorsque $R_3$ désigne un groupe cyanoéthyle, $R_1$ etant un groupe 1-cyanoéthyle ou 2-cyanoéthyle.

$X^{\ominus}$ désigne un anion incolore d'un acide organique ou minéral et

n 0 ou 1.

3. Benzimidazoles selon la revendication 1, de formule

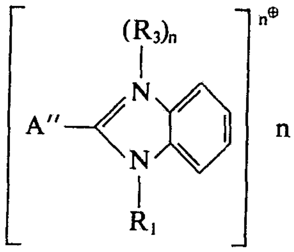 n X⊖ (3)

dans laquelle

A″ désigne le reste

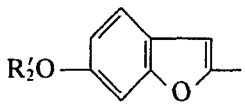

ou lorsque n est le nombre 1, et également le reste,

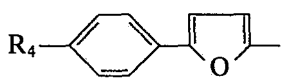

$R_1$ désigne un groupe cyanométhyle, 1-cyanoéthyle ou lorsque n est le nombre 1, également 2-cyanoéthyle ou $C_{3-6}$ carbalkoxyméthyle
$R'_2$ désigne un groupe $C_{1-4}$ alkyle
$R_3$ désigne un groupe cyanométhyle, cyanoéthyle ou $C_{3-6}$ carbalkoxyléthyle, lorsque $R_3$ désigne un groupe cyanoéthyle, $R_2$ étant un groupe 1-cyanoéthyle ou 2-cyanoéthyle
$R_4$ désigne indépendamment l'un de l'autre un atome d'hydrogène ou de chlore
X⊖ désigne un anion incolore d'un acide organique ou mineral et
n 0 ou 1.

4. Benzimidazoles selon la revendication 2, de formule

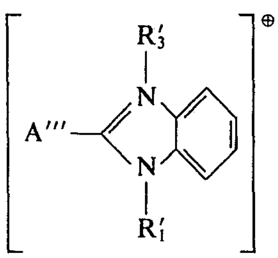 X⊖ (4)

dans laquelle

A‴ désigne le reste

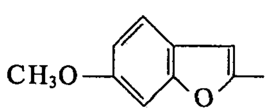 ou 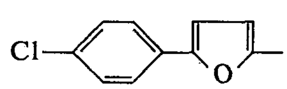

$R'_1$ désigne un groupe cyanométhyle, cyanoéthyle ou $C_{3-6}$ carbalkoxyméthyle,
$R'_3$ désigne un groupe cyanométhyle, cyanoéthyle ou $C_{3-6}$ carbalkoxyméthyle, lorsque $R'_3$ désigne un cyanoéthyle, $R'_1$ est un groupe cyanoéthyle et
X⊖ désigne un anion incolore d'acide organique ou minéral.

5. Benzimidazoles selon la revendication 4, de formule

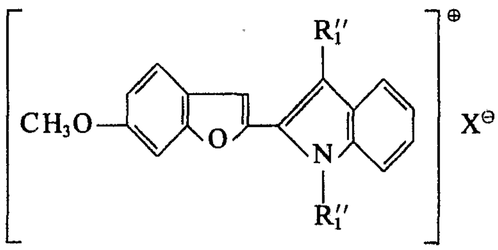

(5)

dans laquelle

$R''_1$ désigne un groupe cyanoéthyle ou $C_{3-4}$ carbalkoxyméthyle et
$X^{\ominus}$ désigne un anion incolore d'un acide minéral ou organique.

6. Benzimidazoles selon la revendication 4, de formule

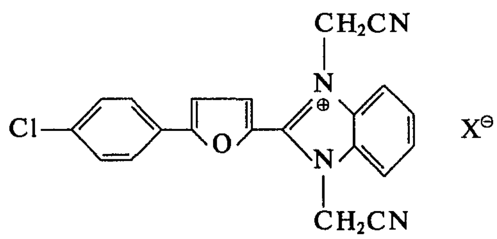

dans laquelle $X^{\ominus}$ désigne un anion incolore d'un acide organique ou minéral.

7. Benzimidazole selon la revendication 3, de formule

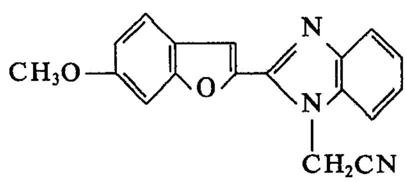

8. Benzimidazoles selon la revendication 5, de formule

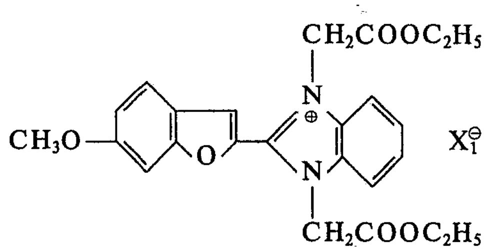

dans laquelle $X_1$ désigne un atome de chlore ou de brome.

9. Benzimidazole selon la revendication 5 de formule

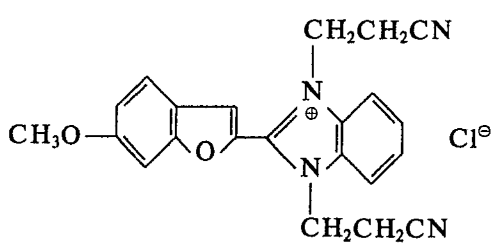

10. Procédé de préparation de benzimidazoles de formule

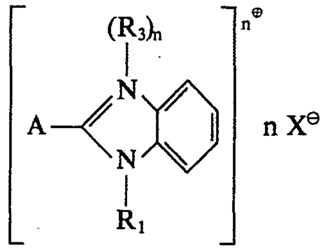

dans laquelle

A désigne le reste

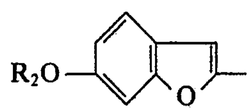

ou

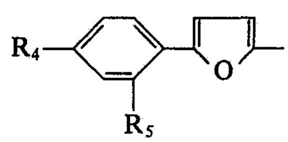

$R_1$ désigne un groupe cyanométhyle, 1-cyanoéthyle ou lorsque n est le nombre 1, également 2-cyanoéthyle ou $C_{3-6}$-carbalkoxyméthyle

$R_2$ désigne un groupe $C_{1-4}$ alkyle ou $C_{3-4}$ Alcényle,

$R_3$ désigne un groupe cyanométhyle, cyanoéthyle ou $C_{3-6}$ carbalkoxyléthyle, lorsque $R_3$ désigne un groupe cyanoéthyle, $R_1$ étant un groupe 1-cyanoéthyle ou 2-cyanoéthyle.

$R_4$ et $R_5$ désignent indépendamment l'un de l'autre un atome d'hydrogène ou de chlore.

$X^{\ominus}$ désigne un anion incolore d'un acide organique ou minéral et

n 0 ou 1,

caractérisé par le fait qu'on convertit un benzimidazole de formule

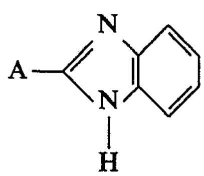

(10)

dans un premier stade en présence d'une base avec au moins un équivalent molaire d'un agent d'alkylation $R_1X$ ou avec l'acrylonitrile en un benzimidazole de formule

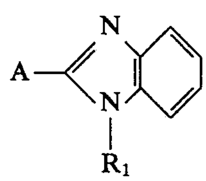

(11)

et éventuellement, on quaternise celui-ci dans une deuxième phase, sans ou avec isolement préalable, avec un composé de formule $R_3X$, dans les formules précitées, A, $R_1$, $R_3$ et X étant définis comme spécifié ci-dessus.

11. Procédé selon la revendication 10, dans lequel on effectue la réaction d'alkylation et la réaction de quaternisation dans un agent d'alkylation en excès ou dans un solvant organique inerte à des températures comprises entre 20° C et 150° C.

12. Procédé de préparation de benzimidazoles de formule

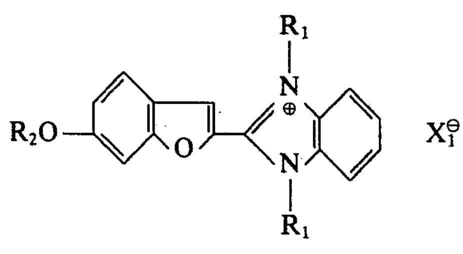

dans laquelle

$R_1$ désigne un groupe cyanométhyle, 1-cyanoéthyle, 2-cyanoéthyle et $C_{3-4}$ carbalkoxyméthyle,
$R_2$ désigne un groupe $C_{1-4}$ alkyle ou $C_{3-4}$ Alcényle et
$X_1$ désigne un atome d'halogène,

caractérisé par le fait que l'on fait réagir, dans un solvant inerte et à des températures comprises entre 20° C et 150° C un halogénure de coumaroyle de formule

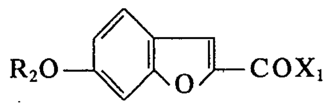

avec une o-phénylènediamine de formule

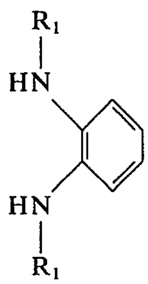

dans lesdites formules $R_1$, $R_2$ et $X_1$ étant définis comme spécifié ci-dessus.

13. Procédé de préparation de benzofurannyl-benzimidazoles de formule

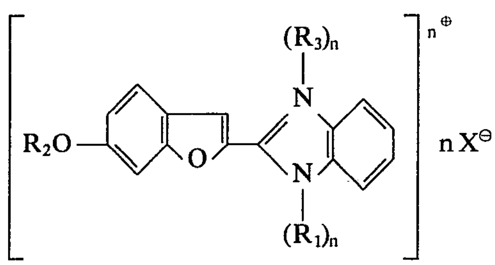

$R_1$ désigne un groupe cyanométhyle, 1-cyanoéthyle ou lorsque n est le nombre 1, également 2-cyanoéthyle ou $C_{3-6}$-carbalkoxyméthyle,
$R_2$ désigne un groupe $C_{1-4}$ alkyle ou $C_{3-4}$ alcényle,
$R_3$ désigne un groupe cyanométhyle, cyanoéthyle ou $C_{3-6}$ carbalkoxyléthyle, lorsque $R_3$ désigne un groupe cyanoéthyle, $R_1$ étant un groupe 1-cyanoéthyle ou 2-cyanoéthyle.
$X^{\ominus}$ désigne un anion incolore d'un acide organique ou minéral et
n 0 ou 1,

caractérisé par le fait que l'on condense n aldéhyde salicylique de formule

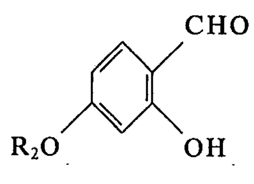

dans laquelle $R_2$ est défini comme spécifié ci-dessus, avec un halogénométhyl-benzimidazole de formule

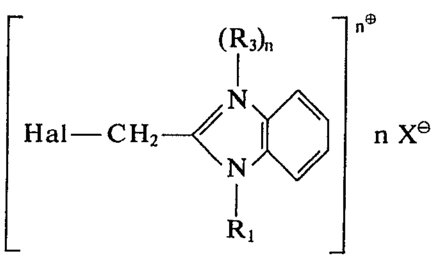

dans laquelle $R_1$, $R_3$, $X^{\ominus}$ et n sont définis comme spécifié ci-dessus et Hal représente un atome de fluor, chlore, ou brome, en présence ou en l'absence d'un agent de condensation basique.

14. Procédé de blanchiement optique, de matières organiques, caractérisé par le fait que l'on incorpore aux matières à blanchir ou que l'on applique sur lesdites matières des benzimidazoles des formules définies dans l'une quelconque des revendications 1 à 9.

15. Procédé selon la revendication 14, caractérisé par le fait que l'on utilise comme matière organique du polyacrylonitrile.

16. Procédé selon la revendication 14, caractérisé par le fait que l'on utilise 0,001 à 2% de l'agent de blanchiement, par rapport au poids de la matière à blanchir.

17. Application des benzimidazoles définis dans l'une quelconque des revendications 1 à 9, comme agents de blanchiement optique pour des matières organiques.

18. Application selon la revendication 17, des benzimidazoles définis dans l'une quelconque des revendications 1 à 9 comme agents de blanchiement optique pour le polyacrylonitrile.

19. Application selon la revendication 17 des benzimidazoles quaternisés définis dans l'une quelconque des revendications 1 à 5, 8 et 9 comme agents de blanchiement optique pour des polyesters modifiés par des groupes acides.

20. Matière organique contenant 0,001 à 2% en poids de benzimidazoles définis dans la revendication 1.